(19) **Europäisches Patentamt / European Patent Office / Office européen des brevets**

(11) **EP 1 782 052 B1**

(12) **EUROPEAN PATENT SPECIFICATION**

(45) Date of publication and mention
of the grant of the patent:
**19.09.2012 Bulletin 2012/38**

(51) Int Cl.:
*G01N 27/407* (2006.01)  *G01N 27/416* (2006.01)
*G01N 33/497* (2006.01)  *G01N 33/00* (2006.01)

(21) Application number: **05773022.8**

(22) Date of filing: **12.08.2005**

(86) International application number:
**PCT/GB2005/003196**

(87) International publication number:
**WO 2006/016188 (16.02.2006 Gazette 2006/07)**

(54) **GAS SENSOR**

GASSENSOR

DÉTECTEUR DE GAZ

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **13.08.2004 GB 0418078**

(43) Date of publication of application:
**09.05.2007 Bulletin 2007/19**

(73) Proprietor: **Anaxsys Technology Limited
Send, Woking
Surrey GU23 7EF (GB)**

(72) Inventors:
• **VARNEY, Mark, Sinclair
Bishopstoke, Hampshire
SO50 8PU (GB)**
• **GARRETT, Michael, Ernest
Woking,
Surrey GU22 7XR (GB)**

(74) Representative: **Icely, Dominic Michael et al
Marks & Clerk LLP
4220 Nash Court
Oxford Business Park South
Oxford OX4 2RU (GB)**

(56) References cited:
EP-A- 0 293 230    WO-A-2004/001407
US-A- 4 298 347    US-A- 4 377 446
US-B1- 6 277 523    US-B1- 6 454 923

• **CREASEY M R ET AL: "The development of a
thick-film electrochemical sensor and
instrumentation for in-situ determination of
carbon dioxide partial pressure (pCO2) in the
marine environment" ELECTRONIC
ENGINEERING IN OCEANOGRAPHY, 1994.,
SIXTH INTERNATIONAL CONFERENCE ON
CAMBRIDGE, UK, LONDON, UK,IEE, UK, 1994,
pages 124-128, XP006512772 ISBN:
0-85296-619-9**
• **PATENT ABSTRACTS OF JAPAN vol. 018, no. 223
(P-1729), 21 April 1994 (1994-04-21) & JP 06
018475 A (MATSUSHITA ELECTRIC WORKS
LTD), 25 January 1994 (1994-01-25)**

**Description**

The present invention is related to a sensor for detecting gaseous substances, in particular a sensor for detecting the presence of substances in a gaseous phase or gas stream. The sensor is particularly suitable for, but not limited to, the detection of carbon dioxide. The sensor finds particular use as a sensor for detecting and measuring the concentration of gases, such as carbon dioxide, in the exhaled breath of a person or animal.

Carbon dioxide can be detected using a variety of analytical techniques and instruments. The most practical and widely used analysers use spectroscopic infra-red absorption as a method of detection, but the gas may also be detected using mass spectrometry, gas chromatography, thermal conductivity and others. Although most analytical instruments, techniques and sensors for carbon dioxide measurement are based on the physicochemical properties of the gas, new techniques are being developed which utilise electrochemistry, and an assortment of electrochemical methods have been proposed. However, it is not possible to measure carbon dioxide ($CO_2$) gas directly using electrochemical techniques. Indirect methods have been devised, based on the dissolution of the gas into an electrolyte with a consequent change in the pH of the electrolyte. Other electrochemical methods use high temperature catalytic reduction of carbon dioxide.

A more recently applied technique is to monitor a specific chemical reaction in an electrolyte that contains suitable organometallic ligands that chemically interact following the pH change induced by the dissolution of the carbon dioxide gas. The pH change then disturbs a series of reactions, and the carbon dioxide concentration in the atmosphere is then estimated indirectly according to the change in the acid-base chemistry.

Carbon dioxide is an acid gas, and interacts with water, and other (protic) solvents. For example, carbon dioxide dissolves in an aqueous solution according to the following reactions:

$$CO_2 + H_2O \Leftrightarrow H_2CO_3 \qquad (1)$$

$$H_2CO_3 \Leftrightarrow HCO_3^- + H^+ \qquad (2)$$

$$HCO_3^- \Leftrightarrow CO_3^{2-} + H^+ \qquad (3)$$

It will be appreciated that, as more carbon dioxide dissolves, the concentration of hydrogen ions ($H^+$) increases.

The use of this technique for sensing carbon dioxide has the disadvantage that when used for gas analysis in the gaseous phase the liquid electrolyte must be bounded by a semi-permeable membrane. The membrane is impermeable to water but permeable to various gases, including carbon dioxide. The membrane must reduce the evaporation of the internal electrolyte without seriously impeding the permeation of the carbon dioxide gas. The result of this construction is an electrode which works well for a short period of time, but in which it has a long response time and the electrolyte needs to be frequently renewed.

WO 04/001407 discloses a sensor comprising a liquid electrolyte retained by a permeable membrane, which overcomes some of these disadvantages. However, it would be very desirable to provide a sensor that does not rely on the presence and maintenance of a liquid electrolyte.

US 4,772,863 discloses a sensor for oxygen and carbon dioxide gases having a plurality of layers comprising an alumina substrate, a reference electrode source of anions, a lower electrical reference electrode of platinum coupled to the reference source of anions, a solid electrolyte containing tungsten and coupled to the lower reference electrode, a buffer layer for preventing the flow of platinum ions into the solid electrolyte and an upper electrode of catalytic platinum.

GB 2,287,543 A discloses a solid electrolyte carbon monoxide sensor having a first cavity formed in a substrate, communicating with a second cavity in which a carbon monoxide adsorbent is located. An electrode detects the partial pressure of oxygen in the carbon monoxide adsorbent. The sensor of GB 2,287,543 is very sensitive to the prevailing temperature and is only able to measure low concentrations of carbon monoxide at low temperatures with any sensitivity. High temperatures are necessary in order to measure carbon monoxide concentrations that are higher, if complete saturation of the sensor is to be avoided. This renders the sensor impractical for measuring gas compositions over a wide range of concentrations.

GB 2,316,178 A discloses a solid electrolyte gas sensor, in which a reference electrode is mounted within a cavity in the electrolyte. A gas sensitive electrode is provided on the outside of the solid electrolyte. The sensor is said to be useful in the detection of carbon dioxide and sulphur dioxide. However, operation of the sensor requires heating to a temperature of at least 200°C, more preferably from 300 to 400°C. This represents a major drawback in the practical applications of the sensor.

Sensors for use in monitoring gas compositions in heat treatment processes are disclosed in GB 2,184,549 A. However, as with the sensors of GB 2,316,178, operation at high temperatures (up to 600°C) is disclosed and appears to be required.

EP-A-0 293 230 discloses an acidic gas sensor having a sensing electrode, a counter electrode and a body of electrolyte containing a complex. The complex has one or more ligands that is displaceable by the presence of the acidic gas in the electrolyte to form a modified complex which can be electrochemically reacted at the sensing electrode.

US 6,454,923 concerns an electrochemical gas sensor. In one embodiment, the sensor comprises a wick which is in close proximity to the electrodes of the sensor and ensures that the electrodes are always in contact with electrolyte.

Accordingly, there is a need for a sensor that does not rely on the presence of an electrolyte in the liquid phase or high temperature catalytic method, that is of simple construction and may be readily applied to monitor gas compositions at ambient conditions. In particular, there is a need for a sensor that can operate at ambient conditions to quickly determine the carbon dioxide content of a person's exhaled breath.

In a first aspect, the present invention provides a sensor for sensing a target substance in a gas stream comprising the target substance and water vapour, the sensor comprising:

a sensing element disposed to be exposed to the gas stream, the sensing element comprising:

a working electrode;
a counter electrode; and
a solid electrolyte precursor extending between and in contact with the working electrode and the counter electrode;
whereby the gas stream may be caused to impinge upon the solid electrolyte precursor such that water vapour in the gas stream at least partially hydrates the precursor to form an electrolyte in electrical contact with the working electrode and the counter electrode.

The sensor is particularly suitable for the detection of carbon dioxide, in particular carbon dioxide present in the exhaled breath of a person or animal.

In the context of the present invention, the term 'solid electrolyte precursor' is a reference to a material that is in the solid phase under the conditions prevailing during the use of the sensor and that can react with (or be hydrated by) water vapour in the gas stream to reconstitute a hydrous electrolyte, allowing current to flow between the working electrode and counter electrode.

The present invention provides a sensor that is compact and of simple construction. In addition, the sensor may be used at ambient temperature conditions, without the need for any heating or cooling, while at the same time producing an accurate measurement of the target substance concentration in the gas being analysed. As the sensor does not employ or rely upon a liquid electrolyte, it provides a long storage and operational lifespan. In addition, the use of a solid electrolyte precursor allows the sensor to be used in a variety of positions, locations and orientations.

The sensor may comprise a passage or conduit to direct the stream of gas onto the solid electrolyte precursor. For example, when the sensor is intended for use in the analysis of the breath of a patient, the conduit may comprise a mouthpiece, into which the patient may exhale.

As noted above, the sensor employs water vapour present in the gas stream to at least partially hydrate the electrolyte precursor to form the electrolyte. In many cases, the gas stream will comprise sufficient water vapour for this to occur. One example is the analysis of exhaled breath from a person or animal. However, the sensor may be provided with a means for increasing the water vapour content of the gas stream, should the water vapour content of the gas be too low. Such means may include a reservoir of water and a dispenser, such as a spray, nebuliser or aerosol.

The electrodes may have any suitable shape and configuration. Suitable forms of electrode include points, lines, rings and flat planar surfaces. The effectiveness of the sensor can depend upon the particular arrangement of the electrodes and may be enhanced in certain embodiments by having a very small path length between the adjacent electrodes. This may be achieved, for example, by having each of the working and counter electrodes comprise a plurality of electrode portions arranged in an alternating, interlocking pattern, that is in the form of an array of interdigitated electrode portions, in particular arranged in a concentric pattern.

The electrodes are preferably oriented as close as possible to each other, to within the printing resolution of the manufacturing technology. The working and counter electrode can be between 10 to 1000 microns in width, preferably from 50 to 500 microns. The gap between the working and counter electrodes can be between 20 and 1000 microns, more preferably from 50 to 500 microns. The optimum track-gap distances are found by routine experiment for the particular electrode material, geometry, configuration, and substrate under consideration. In a preferred embodiment the optimum working electrode track widths are from 50 to 250 microns, preferably about 100 microns, and the counter electrode track widths are from 50 to 750 microns, preferably about 500 microns. The gaps between the working and counter electrodes are preferably about 100 microns.

The counter electrode and working electrode may be of equal size. However, in one preferred embodiment, the surface area of the counter electrode is greater than that of the working electrode to avoid restriction of the current transfer. Preferably, the counter electrode has a surface area at least twice that of the working electrode. Higher ratios of the surface area of the counter electrode and working electrode, such as at least 3:1, preferably at least 5:1 may also be employed. The thickness of the electrodes is determined by the manufacturing technology, but has no direct influence on the electrochemistry. The magnitude of the resultant electrochemical signal is determined principally by exposed surface area, that is the surface area of the electrodes exposed to and in electrical contact with the electrolyte precursor. Generally, an increase in the surface area of the electrodes will result in a higher signal, but may also result in increased susceptibility to noise and electrical interference. However, the signals from smaller electrodes may be more difficult to detect.

The electrodes may be supported on a substrate. Suitable materials for the support substrate are any inert, non-conducting material, for example ceramic, plastic, or glass.

While the sensor operates well with two electrodes, as hereinbefore described, arrangements with more than two electrodes, for example including a third or reference electrode, as is well known in the art. The use of a reference electrode provides for better potentiostatic control of the applied voltage, or the galvanostatic control of current, when the "iR drop" between the counter and working electrodes is substantial. Dual 2-electrode and 3-electrode cells may also be employed.

The electrodes may comprise any suitable metal or alloy of metals, with the proviso that the electrode does not react with the electrolyte or any of the substances present in the gas stream. Preference is given to metals in Group VIII of the Periodic Table of the Elements (as provided in the Handbook of Chemistry and Physics, 62nd edition, 1981 to 1982, Chemical Rubber Company). Preferred Group VIII metals are rhenium, palladium and platinum. Other suitable metals include silver and gold. Preferably, each electrode is prepared from gold or platinum,

The solid electrolyte precursor comprises a ligand, preferably an organic ligand (hereafter denoted as 'L'), which is capable of forming a complex with a metal ion (hereafter denoted as 'M') to form an organometallic complex. Within the electrolyte, the organic ligand is capable of dissociation according to the following equations:

$$LH_2 \Leftrightarrow LH^- + H^+ \qquad (4)$$

$$LH- \Leftrightarrow L^{2-} + H^+ \qquad (5)$$

The increasing concentration of a target substance, such as carbon dioxide gas, within the electrolyte raises the concentration of protons ($H^+$) and also causes the protonation of the ligand. The presence of free dissociated metal ions ($M^{2+}$) also results in a complex with the protonated ligand as follows:

$$M^{2+}+LH_2 \Leftrightarrow LM+2H^+ \qquad (6)$$

The dissociated protons from the dissolution of the target substance interact in all of the above reactions. The quantity of the target substance that has dissolved in the electrolyte can therefore be followed by measuring the quantity of the metal ion that is not complexed with the ligand. Overall, the concentration of the target substance being dissolved in the electrolyte is directly related to the concentration of free metal ions in the electrolyte. The change in concentration of the target substance is also related to the observed change in concentration of the metal ion, although the relationship is not linear.

The chemical species interact with each other in specific ways according to their equilibrium constants. The precise relationship between the concentration of the target substance and the free metal ion concentration is complex and can be theoretically modeled in ways known per se by skilled electrochemists using common general knowledge in the art. The modeling generally involves developing an algorithm which considers each step in the above reactions as a series of "competitive" equilibria.

A wide range of ligands and metal ions may be employed in the organometallic complex of the solid electrolyte precursor. Preferred organic compounds for use as the ligand are amines, in particular diamines, such as diaminopropane, and carboxylic acids, especially dicarboxylic acids. The metal ions are preferably ions of Group VIII of the Periodic Table of the Elements (as provided in the Handbook of Chemistry and Physics, 62nd edition, 1981 to 1982, Chemical Rubber Company). Suitable metals include copper, lead and cadmium.

The specific choice and combination of metal ions and organic ligands may be theoretically calculated using principles of equilibrium (speciation) chemistry. The principle determinand is that the ligand should have a low $pK_b$. As noted above, a preferred class of ligand is the diamines, for example, propanediamine, ethylenediamine and various substituted diamines, The performance of the sensor is dependant on the choice and concentration of metal/ligand pairs and the optimum precursor composition may be found by routine experimentation.

The solid electrolyte precursor preferably also comprises a salt to aid ionic conduction. Metal halide salts are preferred, in particular sodium and potassium halides, especially chlorides.

A particularly preferred composition for the solid electrolyte precursor comprises copper, propanediamine and potassium chloride. One preferred composition has these components present in the following amounts: 4mM copper, 10mM propanediamine, and 0.1M potassium chloride as base electrolyte.

It will be appreciated by those skilled in the art that there are a considerable range and combination of other potential metals, ligands, and base electrolytes.

The solid electrolyte precursor may be prepared from a solution of the constituent components in a suitable solvent. Water is a most convenient solvent. The solvent is removed by drying and evaporation, to leave the solid electrolyte precursor. Evaporation of the solvent may be assisted by blowing a gas stream, such as air or nitrogen, across the surface of the drying precursor.

The solid electrolyte precursor and the electrodes may be combined in any convenient arrangement, provided that the portion of the electrolyte precursor that is hydrated by water vapour in the gas stream is able to bridge the two electrodes and be electrically connected to each of them. In a preferred arrangement, the solid electrolyte precursor is preferably deposited on the electrodes. This may be achieved by conventional techniques, with thick film screen printing being a particularly preferred technique.

Thick film screen printing techniques are known in the art for depositing films of various materials in the processing of microelectronic circuits and a particularly suitable for the preparation of the sensing element in the sensor of the present invention. Screen printing is the transfer of pseudoplastic pastes or inks through a fabric screen onto a substrate. Pseudoplastic pastes have the characteristic of decreasing viscosity with increasing rates of applied shear and are generally applied using a squeegee. The transfer of the ink occurs when contact is made with the surface of the substrate. The high shear generated in the ink as a result of the action of the squeegee passing over the screen results in the ink being pulled through it. The ink is deposited in a pattern defined by the open areas in the emulsion of the screen.

The electrodes of the sensor of the present invention may be formed by printing the electrode material in the form of a thick film screen printing ink onto the substrate. The ink consists of four components, namely the functional component, a binder, a vehicle and one or more modifiers. In the case of the present invention, the functional component forms the conductive component of the electrode and comprises a powder of one or more of the aforementioned metals used to form the electrode.

The binder holds the ink to the substrate and merges with the substrate during high temperature firing. The vehicle acts as the carrier for the powders and comprises both volatile components, such as solvents and non-volatile components, such as polymers. These materials are lost during the early stages of drying and firing respectively. The modifiers comprise small amounts of additives, which are active in controlling the behaviour of the inks before and after processing.

Screen printing requires the ink viscosity to be controlled within limits determined by rheological properties, such as the amount of vehicle components and powders in the ink, as well as aspects of the environment, such as ambient temperature.

The printing screen may be prepared by stretching stainless steel wire mesh cloth across the screen frame, while maintaining high tension. An emulsion is then spread over the entire mesh, filling all open areas of the mesh. A common practice is to add an excess of the emulsion to the mesh. The area to be screen printed is then patterned on the screen using the desired electrode design template.

The squeegee is used to spread the ink over the screen. The shearing action of the squeegee results in a reduction in the viscosity of the ink, allowing the ink to pass through the patterned areas onto the substrate. The screen peels away as the squeegee passes. The ink viscosity recovers to its original state and results in a well defined print. The screen mesh is critical when determining the desired thick film print thickness, and hence the thickness of the completed electrodes.

The mechanical limit to downward travel of the squeegee (downstop) should be set to allow the limit of print stroke to be 75 - 125um below the substrate surface. This will allow a consistent print thickness to be achieved across the substrate whilst simultaneously protecting the screen mesh from distortion and possible plastic deformation due to excessive pressure.

To determine the print thickness the following equation can be used:

$$Tw = (Tm \times Ao) + Te$$

Where Tw = Wet thickness (um);
Tm = mesh weave thickness (um);
Ao = % open area;
Te = Emulsion thickness (um).

After the printing process the sensor element needs to be levelled before firing. The levelling permits mesh marks to fill and some of the more volatile solvents to evaporate slowly at room temperature. If all of the solvent is not removed in this drying process, the remaining amount may cause problems in the firing process by polluting the atmosphere surrounding the sensor element. Most of the solvents used in thick film technology can be completely removed in an oven at 150 C when held there for 10 minutes.

Firing is typically accomplished in a belt furnace. Firing temperatures vary according to the ink chemistry. Most commercially available systems fire at 850 °C peak for 10 minutes. Total furnace time is 30 to 45 minutes, including the time taken to heat the furnace and cool to room temperature. Purity of the firing atmosphere is critical to successful processing. The air should be clean of particulates, hydrocarbons, halogen-containing vapours and water vapour.

In a further aspect, the present invention provides a method of sensing a target substance in a gas stream comprising:

causing a gas stream comprising the target substance and water vapour to impinge upon a solid electrolyte precursor;
allowing the solid electrolyte precursor to at least partially hydrate, so as to form an electrolyte bridge between a working electrode and a counter electrode;
applying a electric potential across the working electrode and counter electrode;
measuring the current flowing between the working electrode and counter electrode as a result of the applied potential; and
determining from the measured current flow an indication of the concentration of the target substance in the gas stream.

As noted above, the method of the present invention is particularly suitable for use in the detection of carbon dioxide in a gas stream.

The method of the present invention may be carried out using a sensor as hereinbefore described.

As noted above, the method relies upon the presence of water vapour in the gas stream to hydrate at least a portion of the solid electrolyte precursor, to provide the sensor with an electrolyte bridging the electrodes. Should the gas stream contain too little water vapour for the required level of hydration of the precursor to be achieved, additional water may be added to the gas before contact with the precursor takes place.

The method requires that an electric potential is applied across the electrodes. In one simple configuration, a voltage is applied to the counter electrode, while the working electrode is connected to earth (grounded). In its simplest form, the method applies a single, constant potential difference across the working and counter electrodes. Alternatively, the potential difference may be varied against time. In one embodiment, the electric potential is pulsed between a so-called 'rest' potential, at which no reaction with the metal ions occurs, and a reaction potential.

The measured current in the sensor element is usually small. The current is converted to a voltage using a resistor, R. As a result of the small current flow, careful attention to electronic design and detail may be necessary. In particular, special "guarding" techniques may be employed. Ground loops need to be avoided in the system. This can be achieved using techniques known in the art.

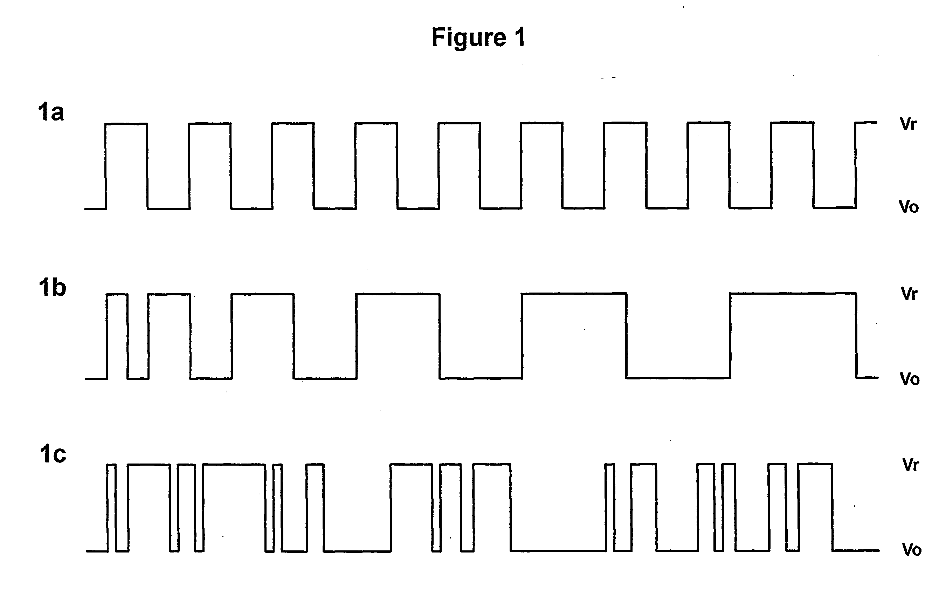

The potential difference applied to the electrodes of the sensor element may alternate or be periodically pulsed between a rest potential and a reaction potential, as noted above. Figure 1 shows examples of voltage forms that may be applied. Figure 1a is a representation of a pulsed voltage signal, alternating between a rest potential, $V_0$, and a reaction potential $V_R$. The voltage may be pulsed at a range of frequencies, typically from sub-Hertz frequencies, that is from 0.1 Hz, to from 2 to 10kHz. A preferred pulse frequency is in the range of from 1 to 500 Hz. Alternatively, the potential waveform applied to the counter electrode may consist of a "swept" series of frequencies, represented in Figure 1b. A further alternative waveform shown in Figure 1c is a so-called "white noise" set of frequencies. The complex frequency response obtained from such a waveform will have to be deconvoluted after signal acquisition using techniques such as Fourier Transform analysis. Again, such techniques are known in the art.

The shape of the transient response can be simply related to the electrical characteristics (impedance) of the sensor in terms of resistance and capacitance. By careful analysis, the individual contributions of resistance and capacitance may be calculated. In a preferred embodiment, the sensor uses an electrochemical technique, known as square wave voltammetry (SWV).

One preferred voltage regime is 0V ("rest" potential), 250mV ("reaction" potential), and 20Hz pulse frequency.

It is an advantage of the present invention that the electrochemical reaction potential is approximately +0.2 volts, which avoids many if not all of the possible competing reactions that would interfere with the measurements, such as the reduction of metal ions and the dissolution of oxygen.

The method of the present invention is particularly suitable for use in the analysis of the exhaled breath of a person or animal. From the results of this analysis, an indication of the respiratory condition of the patient may be obtained.

Accordingly, in a further aspect, the present invention provides a method of measuring the concentration of a target substance in the exhaled breath of a patient, the method comprising:

causing the exhaled breath to impinge upon a solid electrolyte precursor;
allowing the solid electrolyte precursor to at least partially hydrate, so as to form an electrolyte bridge between a working electrode and a counter electrode;

applying a electric potential across the working electrode and counter electrode;

measuring the current flowing between the working electrode and counter electrode as a result of the applied potential; and

determining from the measured current flow an indication of the concentration of the target substance in the exhaled breath stream.

The gas exhaled by a person or animal is saturated in water vapour, as a result of the action of the gas exchange mechanisms taking place in the lungs of the subject. As noted above, at least a portion of the solid electrolyte precursor is caused to dissolve by water vapour in the breath being exhaled by the patient. This in turn allows the target substance, such as carbon dioxide, in the gas stream to dissolve and interact with the metal-ligand species, as described hereinbefore.

The sensor and method of the present invention are particularly suitable for analyzing tidal concentrations of substances, such as carbon dioxide, in the exhaled breath of a person, to diagnose or monitor a variety of respiratory conditions. The sensor is particularly useful for applications requiring fast response times, for example personal respiratory monitoring of tidal breathing (capnography). Capnographic measurements can be applied generally in the field of respiratory medicine, airway diseases, both restrictive and obstructive, airway tract disease management, and airway inflammation. The present invention finds particular application in the field of capnography and asthma diagnosis, monitoring and management, where the shape of the capnogram changes as a function of the extent of the disease. In particular, due to the high rate of response that may be achieved using the sensor and method of the present invention, the results may be used to provide an early alert to the onset of an asthma attack in an asthmatic patient.

Embodiments of the present invention will now be described, by way of example only, having reference to the accompanying drawings, in which:

Figures 1a, 1b and 1c are voltage vs. time representations of possible voltage waveforms that may be applied to the electrodes in the method of the present invention, as discussed hereinbefore;

Figure 2 is a representation of one embodiment of the sensor of the present invention. The tubing adaptor is "cut-away" to reveal the relative position of the sensor within the interior;

Figure 3 is an isometric schematic view of a face of one embodiment of the sensor element according to the present invention;

Figure 4 is an isometric schematic view of an alternative embodiment of the sensor element of the sensor of the present invention;

Figure 5 is a schematic view of a potentiostat electronic circuit that may be used to excite the electrodes of the sensor element;

Figure 6 is a schematic view of a galvanostat electronic circuit that may be used to excite the electrodes;

Figure 7 is a schematic representation of a breathing tube adaptor for use in the sensor of the present invention;

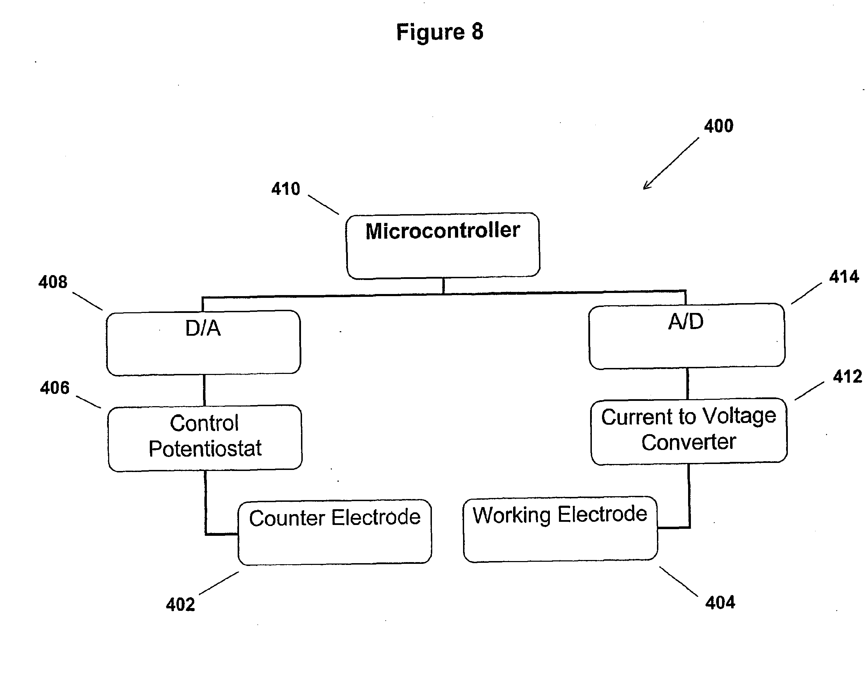

Figure 8 is a flow-diagram providing an overview of the inter-connection of sensor elements and their connection into a suitable measuring instrument of an embodiment of the present invention;

Figure 9 is a typical output recorded by a sensor according to the present invention, showing the response versus time in the analysis of a humidified stream of carbon dioxide; and

Figure 10 is a typical output recorded by a sensor according to the present invention, showing the response versus time in the analysis of carbon dioxide present in the exhaled breath of a patient.

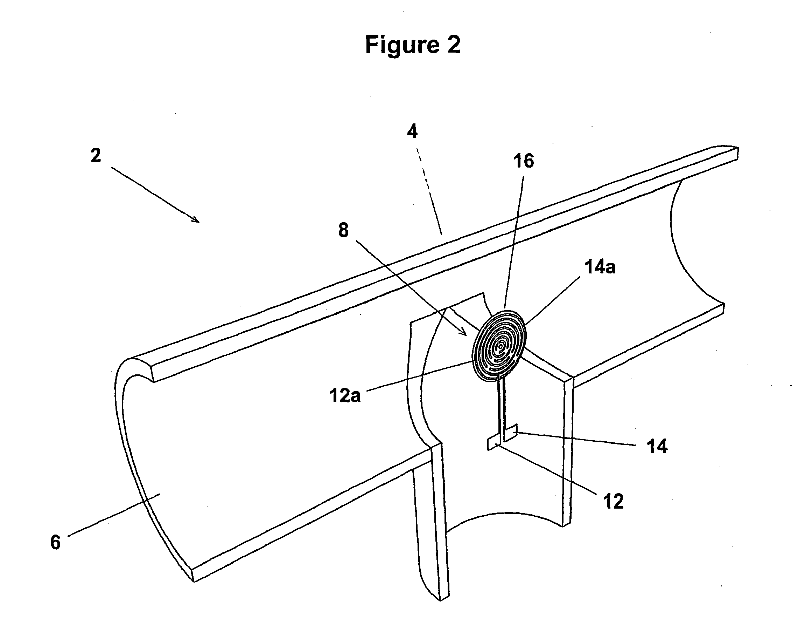

Referring to Figure 2, there is shown a sensor according to the present invention. The sensor is for analyzing the carbon dioxide content of exhaled breath. The sensor, generally indicated as 2, comprises a conduit 4, through which a stream of exhaled breath may be passed. The conduit 4 comprises a mouthpiece 6, into which the patient may breathe.

A sensing element, generally indicated as 8, is located within the conduit 4, such that a stream of gas passing through the conduit from the mouthpiece 6 is caused to impinge upon the sensing element 8. The sensing element 8 comprises a support substrate 10 of an inert material, onto which is mounted a working electrode 12 and a counter electrode 14. The working electrode 12 and reference electrode 14 each comprise a plurality of electrode portions, 12a and 14a, arranged in concentric circles, so as to provide an interwoven pattern minimizing the distance between adjacent portions of the working electrode 12 and reference electrode 14. In this way, the current path between the two electrodes is kept to a minimum.

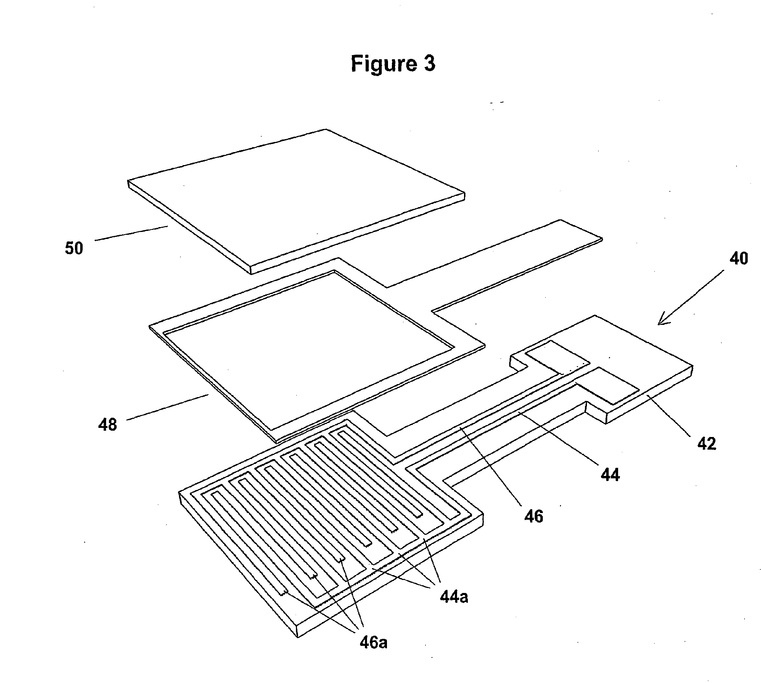

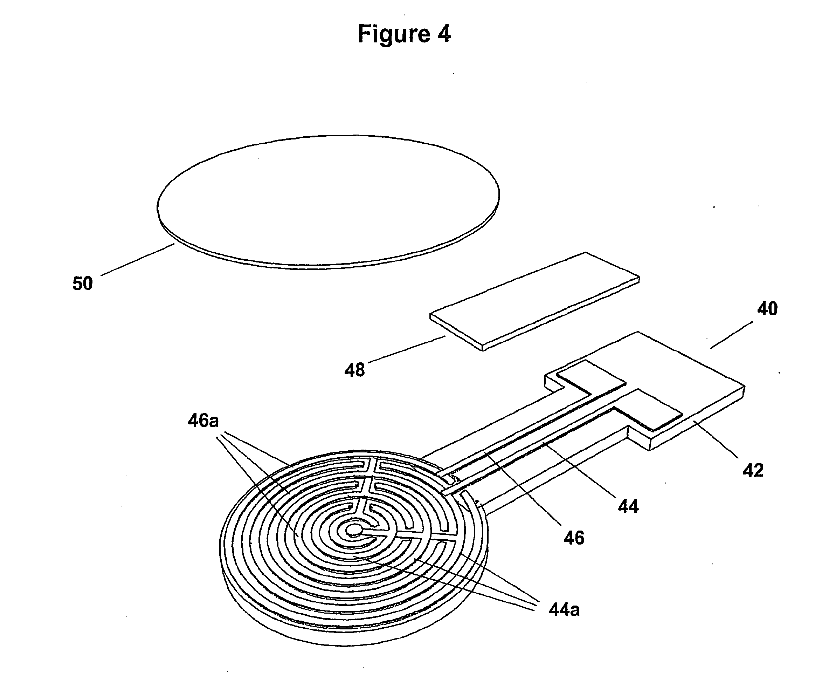

A solid electrolyte precursor 16 is disposed on the working electrode 12 and reference electrode 14. The arrangement of the support, electrodes 12 and 14, and the solid electrolyte precursor is shown in more detail in Figures 3 and 4.

Referring to Figure 3, there is shown an exploded view of a sensor element, generally indicated as 40, comprising a substrate layer 42. A working electrode 44 is mounted on the substrate layer 42 from which extend a series of elongated electrode portions 44a. Similarly, a reference electrode 46 is mounted on the substrate layer 42 from which extends a series of electrode portions 46a. As will be seen in Figure 3, the working electrode portions 44a and the reference electrode portions 46a extend one between the other in an intimate, interdigitated array, providing a large surface area of exposed electrode with minimum separation between adjacent portions of the working and reference electrodes. A layer of dielectric material 48 overlies the working and reference electrodes 44, 46. A layer of electrolyte precursor 50 overlies the layer of dielectric material 48 and the electrode portions 44a and 46a left exposed by the dielectric layer. The electrolyte precursor 50 is in intimate, electrical contact with the portions 44a and 46a of the working and reference electrodes.

An alternative electrode arrangement is shown in Figure 4, in which components common to the sensor element of Figure 3 are identified with the same reference numerals. It will be noted that the working electrode portions 44a and the reference electrode portions 46a are arranged in an intimate circular array.

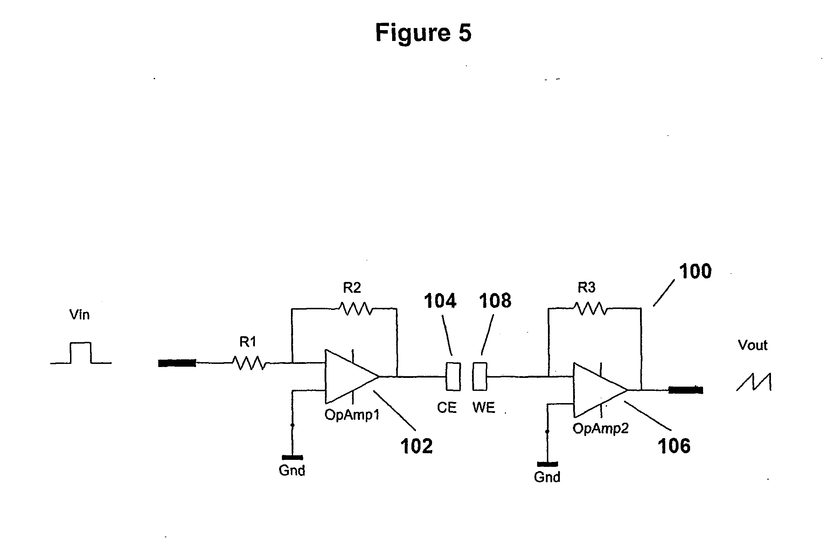

Referring to Figure 5, there is shown a potentiostat electronic circuit that may be employed to provide the voltage applied across the working and reference electrodes of the sensor of the present invention. The circuit, generally indicated as 100, comprises an amplifier 102, identified as 'OpAmp1', acting as a control amplifier to accept an externally applied voltage signal $V_{in}$. The output from OpAmp1 is applied to the control (counter) electrode 104. A second amplifier 106, identified as 'OpAmp2' converts the passage of current from the counter electrode 104 to the working electrode 108 into a measurable voltage ($V_{out}$). Resistors R1, R2 and R3 are selected according to the input voltage, and measured current.

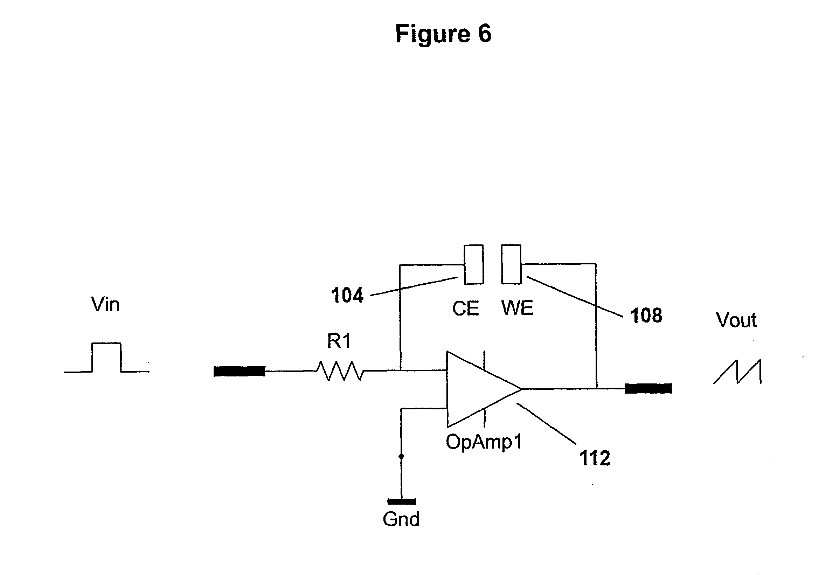

An alternative galvanostat circuit for exciting the electrodes of the sensor is shown in Figure 6. The control and working electrodes 104 and 108 are connected between the input and output of a single amplifier 112, indicated as 'OpAmp1'. Again, resistor R1 is selected according to desired current.

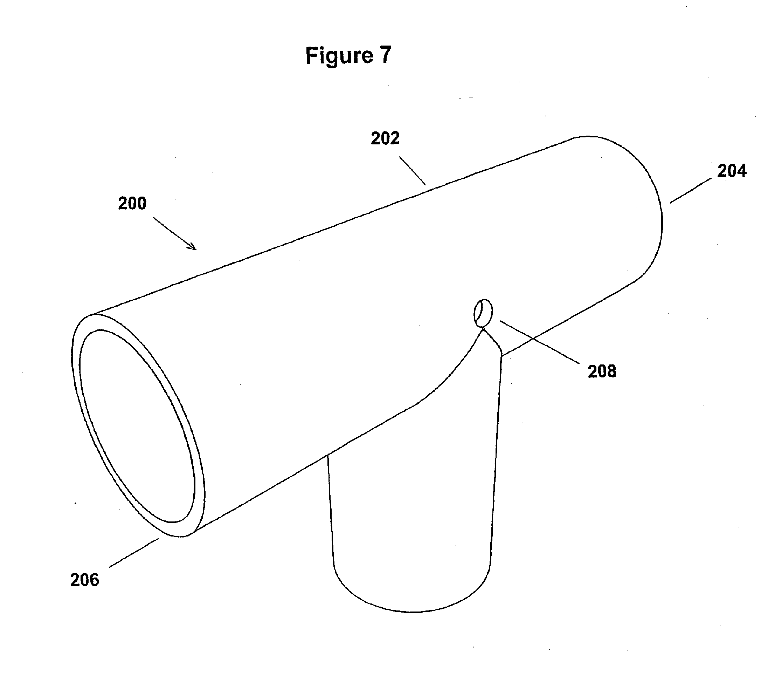

Turning to Figure 7, an adaptor for monitoring the breath of a patient is shown. A sensor element is mounted within the adaptor and oriented directly into the air stream flowing through the adaptor, in a similar manner to that shown in Figure 2 and described hereinbefore. The preferred embodiment illustrated in Figure 7 comprises and adaptor, generally indicated as 200, having a cylindrical housing 202 having a male-shaped (push-fit) cone coupling 204 at one end and a female-shaped (push-fit) cone coupling 206 at the other. There is a small orifice (208) directly adjacent to the sensor.

The sensors of the present invention may be employed individually, or as a series of sensor elements connected sequentially together in-line to measure a series of gases from a single gas stream. For example, a series of sensors may be employed to analyse the exhaled breath of a patient. In addition, two or more sensors may be used to compare the composition of the inhaled and exhaled breath of a patient.

EXAMPLES

The sensor and method of the present invention are further illustrated by the following working examples.

Example 1

A sensor element was prepared comprising gold working and reference electrodes supported on an alumina substrate. The electrodes were applied to the substrate using the screen printing method detailed hereinbefore. The electrodes were arranged as shown in Figure 4.

An aqueous solution containing 4mM copper sulphate and 10mM propanediamine was applied to the polished electrodes by means of a syringe, after which the solution was evaporated to dryness by natural convection, to form the electrolyte precursor.

The sensor was supported by a clamp stand and was exposed on all sides to the ambient atmosphere. Carbon dioxide gas (99.99% purity, ex. BOC Limited) was bubbled at a flow rate of 10 litres per minute through deionised water retained in a vertically-mounted column of 1 cm diameter and 10cm length at a temperature of 38°C to saturate and equilibrate the gas.

A D/A was used to apply successive voltages of 0V and 250mV at a frequency of 0.055 seconds per pulse (18 Hz square wave cycle) across the working and counter electrodes of the sensor. The current response was converted to a measurable voltage by an A/D converter, controlled by a microcontroller.

The humidified stream of carbon dioxide gas was directed at the sensor from a nozzle placed 1 cm from the sensor element. The gas stream was applied to the sensor element for a period of 60 seconds, in order to determine the response of the sensor element and the change in the signal.

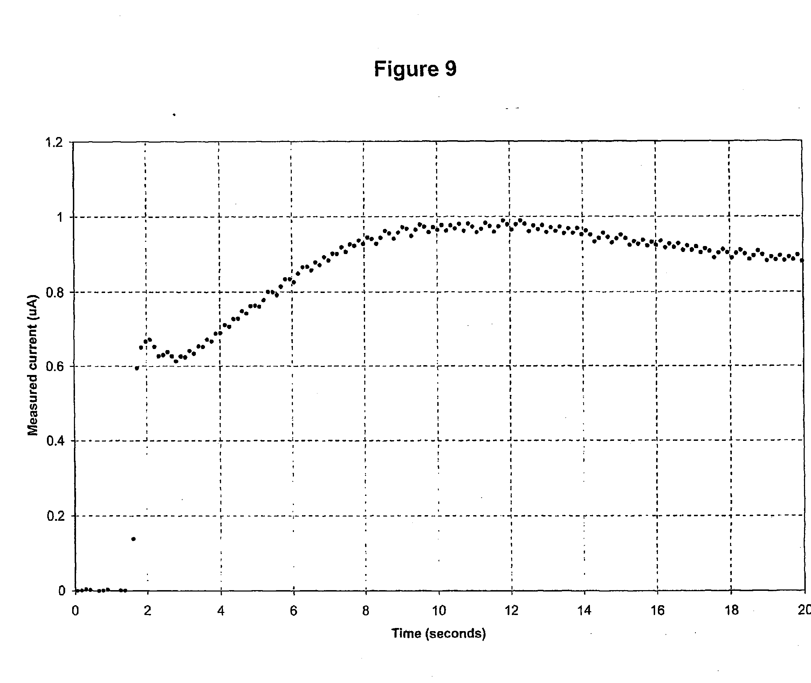

The response of the sensor element is shown graphically in Figure 9, in which the measured output current (microAmps) is plotted against time (seconds). It will be noted that the sensor responded very rapidly to the change in carbon dioxide concentration.

Example 2

A sensor element was prepared as described in Example 1.

The sensor element was housed in a T-piece adaptor, of the type shown in Figure 7, so as to be positioned directly in the air stream passing from the inlet to the outlet of the T-piece. The adaptor was modified as follows to allow the tidal breathing of a patient to be analysed. The adaptor was fitted with a one-way valve at its outlet. A side inlet in the form of a 2mm diameter hole was formed in the housing adjacent the sensor element, so as to direct inhaled gases over the electrode.

A voltage was applied to the electrodes of the sensor, as described in Example 1 and having the wave form described in Example 1.

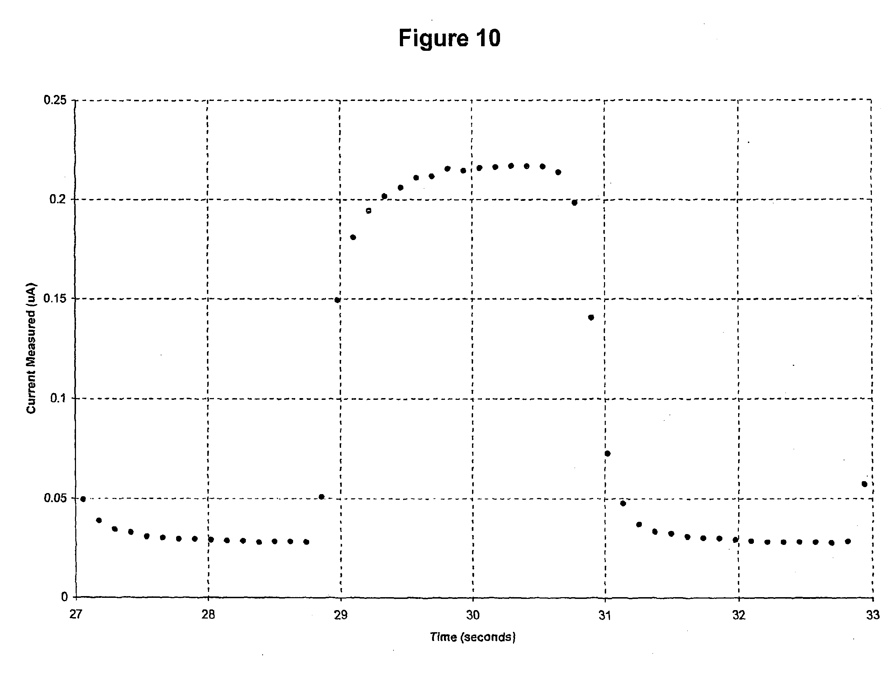

The response of the sensor element was recorded and is shown graphically in Figure 10, in which the measured current (microAmps) is plotted against time. The graph represents the change in concentration of carbon dioxide in the breath over time (capnogram). Due to the very fast response of the sensor, a succession of between 10 and 20 capnograms can be recorded within a total of 60 seconds.

**Claims**

1. A sensor for sensing a target substance in a gas stream comprising the target substance and water vapour, the sensor comprising:

    a sensing element disposed to be exposed to the gas stream, the sensing element comprising:

        a working electrode (12);
        a counter electrode (14) and
        a solid electrolyte precursor (16), being an organic ligand and a metal ion capable of together forming an organometallic complex, extending between and in contact with the working electrode and the counter electrode;
        whereby the gas stream may be caused to impinge upon the solid electrolyte precursor such that water vapour In the gas stream at least partially hydrates the precursor to form an electrolyte in electrical contact with the working electrode and the counter electrode.

2. The sensor according to claim 1, wherein the target substance is carbon dioxide.

3. The sensor according to claim 1 or 2, further comprising a conduit through which the gas stream is channeled to impinge directly upon the sensing element.

4. The sensor according to claim 3, wherein the conduit comprises a mouthpiece into which a patient may exhale.

5. The sensor according to any preceding claim, wherein the working electrode and counter electrode are in a form selected from a point, a line, rings and flat planar surfaces.

6. The sensor according to any preceding claim, wherein one or both of the working electrode and the counter electrode comprises a plurality of electrode portions.

7. The sensor according to claim 6, wherein both the working electrode and the counter electrode comprise a plurality of electrode portions arranged in an interlocking pattern.

8. The sensor according to claim 7, wherein the electrode portions are arranged in a concentric pattern.

9. The sensor according to any preceding claim, wherein the surface area of the counter electrode is greater than the surface area of the working electrode.

10. The sensor according to claim 9, wherein the ratio of the surface area of the counter electrode to the working electrode is at least 2:1,

11. The sensor according to any preceding claim, wherein the electrodes are supported on an inert substrate.

12. The sensor according to any preceding claim, wherein each electrode comprises a metal selected from Group VIII of the Periodic Table of the Elements, copper, silver and gold, preferably gold or platinum.

13. The sensor according to any preceding claim, wherein the solid electrolyte precursor comprises a ligand selected from diamines and dicarboxylic acids.

14. The sensor according to any preceding claim, wherein the solid electrolyte precursor comprises a metal selected from Group VIII of the Periodic Table of the Elements, copper, lead and cadmium.

15. The sensor according to any preceding claim, wherein the solid electrolyte precursor comprises a salt, preferably a metal halide.

16. The sensor according to any preceding claim, wherein the solid electrolyte precursor is applied directly to each electrode, preferably by thick film screen or Inkjet printing technologies.

17. A methods of sensing a target substance in a gas stream comprising:

    causing a gas stream comprising the target substance and water vapour to impinge upon a solid electrolyte precursor, being an organic ligand and a metal ion capable of together forming an organometallic complex allowing the solid electrolyte precursor to at least partially hydrate, so as to form an electrolyte bridge between a working electrode and a counter electrode;
    applying an electric potential across the working electrode and counter electrode;
    measuring the current flowing between the working electrode and counter electrode as a result of the applied potential; and
    determining from the measured current flow an indication of the concentration of the target substance in the gas stream.

18. The method of claim 17, wherein the target substance is carbon dioxide.

19. The method of claim 17 or 18, wherein a constant voltage is applied across the working electrode and the counter electrode.

20. The method of claim 17 or 18, wherein a variable voltage is applied across the working electrode and the counter electrode.

21. The method of claim 20, wherein the variable voltage alternates between a rest potential and a potential above the reaction threshold potential.

22. The method of claim 21, wherein the voltage is pulsed at a frequency of from 0.1Hz to 20 kHz.

23. A method of measuring the concentration of a target substance in the exhaled breath of a patient, the method comprising:

    causing the exhaled breath to impinge upon a solid electrolyte precursor being an organic ligand and metal ion capable of together forming an organometallic complex ;
    allowing the solid electrolyte precursor to at least partially hydrate, so as to form an electrolyte bridge between a working electrode and a counter electrode;
    applying a electric potential across the working electrode and counter electrode;
    measuring the current flowing between the working electrode and counter electrode as a result of the applied potential, and
    determining from the measured current flow an indication of the concentration of a target substance in the exhaled breath stream.

**24.** The method of claim 23, wherein the target substance is carbon dioxide.

**25.** The method of claim 23 or 24, wherein the method is applied to a patient suffering from asthma.

**26.** The method of any of claims 23 to 25, wherein the tidal breathing of a patient is monitored.

**27.** A system for monitoring the composition of a gas stream comprising,

a sensor according to any of claims 1 to 16;
a microcontroller for receiving an output from the sensor; and a display; wherein
the microcontroller is programmed to generate a continuous image of the concentration of a target substance in a gas stream being analysed on the display.

**28.** The system of claim 27, wherein the sensor is adapted to be exposed to the breath of a patient.

**29.** The system of claim 27 or 28, wherein the target substance is carbon dioxide.


**Patentansprüche**

**1.** Sensor zum Erfassen einer Zielsubstanz in einem Gasstrom, der die Zielsubstanz und Wasserdampf enthält, wobei der Sensor Folgendes umfasst:

ein Erfassungselement, das so angeordnet ist, dass es dem Gasstrom ausgesetzt wird, wobei das Erfassungselement Folgendes umfasst:

eine Arbeitselektrode (12);
eine Gegenelektrode (14); und
einen festen Elektrolyt-Vorläufer (16), der ein organischer Ligand und ein Metallion ist, die zusammen einen metallorganischen Komplex bilden können, der zwischen und in Kontakt mit der Arbeitselektrode und der Gegenelektrode verläuft;
wobei bewirkt werden kann, dass der Gasstrom auf den festen Elektrolyt-Vorläufer auftrifft, so dass Wasserdampf im Gasstrom den Vorläufer wenigstens teilweise hydriert, um einen Elektrolyt in elektrischem Kontakt mit der Arbeitselektrode und der Gegenelektrode zu bilden.

**2.** Sensor nach Anspruch 1, wobei die Zielsubstanz Kohlendioxid ist.

**3.** Sensor nach Anspruch 1 oder 2, der ferner eine Leitung beinhaltet, durch die der Gasstrom so geführt wird, dass er direkt auf das Erfassungselement auftrifft.

**4.** Sensor nach Anspruch 3, wobei die Leitung ein Mundstück aufweist, in das ein Patient ausatmen kann.

**5.** Sensor nach einem der vorherigen Ansprüche, wobei die Arbeitselektrode und die Gegenelektrode in einer Form vorliegen, die aus einem Punkt, einer Linie, Ringen und flachen ebenen Oberflächen ausgewählt ist.

**6.** Sensor nach einem der vorherigen Ansprüche, wobei die Arbeitselektrode und/oder die Gegenelektrode mehrere Elektrodenabschnitte umfasst/umfassen.

**7.** Sensor nach Anspruch 6, wobei sowohl die Arbeitselektrode als auch die Gegenelektrode mehrere Elektrodenabschnitte aufweisen, die in einem ineinandergreifenden Muster angeordnet sind.

**8.** Sensor nach Anspruch 7, wobei die Elektrodenabschnitte in einem konzentrischen Muster angeordnet sind.

**9.** Sensor nach einem der vorherigen Ansprüche, wobei der Oberflächeninhalt der Gegenelektrode größer ist als der Oberflächeninhalt der Arbeitselektrode.

**10.** Sensor nach Anspruch 9, wobei das Verhältnis zwischen dem Oberflächeninhalt der Gegenelektrode und dem der Arbeitselektrode wenigstens 2:1 beträgt.

11. Sensor nach einem der vorherigen Ansprüche, wobei die Elektroden auf einem inerten Substrat gelagert sind.

12. Sensor nach einem der vorherigen Ansprüche, wobei jede Elektrode ein Metall umfasst, das ausgewählt ist aus der Gruppe VIII des Periodensystems der Elemente, Kupfer, Silber und Gold, vorzugsweise Gold oder Platin.

13. Sensor nach einem der vorherigen Ansprüche, wobei der feste Elektrolyt-Vorläufer einen Liganden umfasst, der aus Diaminen und Dicarbonsäuren ausgewählt ist.

14. Sensor nach einem der vorherigen Ansprüche, wobei der feste Elektrolyt-Vorläufer ein Metall umfasst, das ausgewählt ist aus der Gruppe VIII des Periodensystems der Elemente, Kupfer, Blei und Cadmium.

15. Sensor nach einem der vorherigen Ansprüche, wobei der feste Elektrolyt-Vorläufer ein Salz, vorzugsweise ein Metallhalid umfasst.

16. Sensor nach einem der vorherigen Ansprüche, wobei der feste Elektrolyt-Vorläufer direkt auf jede Elektrode aufgebracht wird, vorzugsweise durch Dickfilmsiebdruck- oder Tintenstrahldrucktechniken.

17. Verfahren zum Erfassen einer Zielsubstanz in einem Gasstrom, das Folgendes beinhaltet:

Bewirken, dass ein Gasstrom, der die Zielsubstanz und Wasserdampf enthält, auf einen festen Elektrolyt-Vorläufer auftrifft, der ein organischer Ligand und ein Metallion ist, die zusammen einen metallorganischen Komplex bilden können;
Zulassen, dass der feste Elektrolyt-Vorläufer wenigstens teilweise hydriert, um eine Elektrolytbrücke zwischen einer Arbeitselektrode und einer Gegenelektrode zu bilden;
Anlegen eines elektrischen Potentials an die Arbeitselektrode und die Gegenelektrode;
Messen des Stroms, der infolge des angelegten Potentials zwischen der Arbeitselektrode und der Gegenelektrode fließt; und
Ermitteln einer Anzeige für die Konzentration der Zielsubstanz im Gasstrom anhand des gemessenen Stromflusses.

18. Verfahren nach Anspruch 17, wobei die Zielsubstanz Kohlendioxid ist.

19. Verfahren nach Anspruch 17 oder 18, wobei eine konstante Spannung an die Arbeitselektrode und die Gegenelektrode angelegt wird.

20. Verfahren nach Anspruch 17 oder 18, wobei eine veränderliche Spannung an die Arbeitselektrode und die Gegenelektrode angelegt wird.

21. Verfahren nach Anspruch 20, wobei die veränderliche Spannung zwischen einem Ruhepotential und einem Potential über dem Reaktionsschwellenpotential abwechselt.

22. Verfahren nach Anspruch 21, wobei die Spannung mit einer Frequenz von 0,1 Hz bis 20 kHz pulsiert wird.

23. Verfahren zum Messen der Konzentration einer Zielsubstanz in der Ausatemluft eines Patienten, wobei das Verfahren Folgendes beinhaltet:

Bewirken, dass die Ausatemluft auf einen festen Elektrolyt-Vorläufer auftrifft, der ein organischer Ligand und ein Metallion ist, die zusammen einen metallorganischen Komplex bilden können;
Zulassen, dass der feste Elektrolyt-Vorläufer wenigstens teilweise hydriert, um eine Elektrolytbrücke zwischen einer Arbeitselektrode und einer Gegenelektrode zu bilden;
Anlegen eines elektrischen Potentials an die Arbeitselektrode und die Gegenelektrode;
Messen des Stroms, der infolge des angelegten Potentials zwischen der Arbeitselektrode und der Gegenelektrode fließt; und
Ermitteln einer Anzeige für die Konzentration einer Zielsubstanz in dem ausgeatmeten Luftstrom anhand des gemessenen Stromflusses.

24. Verfahren nach Anspruch 23, wobei die Zielsubstanz Kohlendioxid ist.

**25.** Verfahren nach Anspruch 23 oder 24, wobei das Verfahren auf einen an Asthma leidenden Patienten angewandt wird.

**26.** Verfahren nach einem der Ansprüche 23 bis 25, wobei die Ruheatmung eines Patienten überwacht wird.

**27.** System zum Überwachen der Zusammensetzung eines Gasstroms, das Folgendes umfasst:

einen Sensor nach einem der Ansprüche 1 bis 16;
eine Mikrosteuerung zum Empfangen eines Ausgangs von dem Sensor; und ein Display, wobei
die Mikrosteuerung so programmiert ist, dass sie ein kontinuierliches Bild der Konzentration einer Zielsubstanz
in einem analysierten Gasstrom auf dem Display erzeugt.

**28.** System nach Anspruch 27, wobei der Sensor so gestaltet ist, dass er dem Atem eines Patienten ausgesetzt ist.

**29.** System nach Anspruch 27 oder 28, wobei die Zielsubstanz Kohlendioxid ist.

**Revendications**

**1.** Capteur destiné à détecter une substance cible dans un flux gazeux comprenant la substance cible et de la vapeur d'eau, le capteur comprenant :

un élément de détection disposé de manière à être exposé au flux gazeux, l'élément de détection comprenant :

une électrode active (12) ;
une électrode auxiliaire (14) ; et
un précurseur à électrolyte solide (16) de la forme d'un ligand organique et d'un ion métallique en mesure de former ensemble un complexe organométallique, s'étendant entre et en contact avec l'électrode active et l'électrode auxiliaire ;
moyennant quoi le flux gazeux peut être amené à heurter le précurseur à électrolyte solide, de sorte que de la vapeur d'eau dans le flux gazeux hydrate au moins partiellement le précurseur, en vue de former un électrolyte en contact électrique avec l'électrode active et l'électrode auxiliaire.

**2.** Capteur selon la revendication 1, dans lequel la substance cible est du dioxyde de carbone.

**3.** Capteur selon la revendication 1 ou 2, comprenant en outre un conduit à travers lequel le flux gazeux est acheminé en vue de heurter directement l'élément de détection.

**4.** Capteur selon la revendication 3, dans lequel le conduit comprend un embout buccal dans lequel un patient peut expirer.

**5.** Capteur selon l'une quelconque des revendications précédentes, dans lequel l'électrode active et l'électrode auxiliaire sont sous une forme sélectionnée parmi un point, une ligne, des anneaux et des surfaces planes.

**6.** Capteur selon l'une quelconque des revendications précédentes, dans lequel l'une ou les deux parmi l'électrode active et l'électrode auxiliaire comporte(nt) une pluralité de parties d'électrode.

**7.** Capteur selon la revendication 6, dans lequel l'électrode active et l'électrode auxiliaire comprennent une pluralité de parties d'électrode agencées dans un motif entrecroisé.

**8.** Capteur selon la revendication 7, dans lequel les parties d'électrode sont agencées dans un motif concentrique.

**9.** Capteur selon l'une quelconque des revendications précédentes, dans lequel la surface de l'électrode auxiliaire est supérieure à la surface de l'électrode active.

**10.** Capteur selon la revendication 9, dans lequel le rapport entre la surface de l'électrode auxiliaire et la surface de l'électrode active est d'au moins 2:1.

**11.** Capteur selon l'une quelconque des revendications précédentes, dans lequel les électrodes sont supportées sur un substrat inerte.

**12.** Capteur selon l'une quelconque des revendications précédentes, dans lequel chaque électrode comprend un métal choisi dans le groupe VIII de la classification périodique des éléments, du cuivre, de l'argent et de l'or, de préférence de l'or ou du platine.

**13.** Capteur selon l'une quelconque des revendications précédentes, dans lequel le précurseur à électrolyte solide comprend un ligand sélectionné parmi les diamines et les acides dicarboxyliques.

**14.** Capteur selon l'une quelconque des revendications précédentes, dans lequel le précurseur à électrolyte solide comprend un métal sélectionné dans le groupe VIII de la classification périodique des éléments, du cuivre, du plomb et du cadmium.

**15.** Capteur selon l'une quelconque des revendications précédentes, dans lequel le précurseur à électrolyte solide comprend un sel, de préférence un halogénure métallique.

**16.** Capteur selon l'une quelconque des revendications précédentes, dans lequel le précurseur à électrolyte solide est appliqué directement à chaque électrode, de préférence par des technologies de sérigraphie en couche épaisse ou des technologies d'impression à jet d'encre.

**17.** Procédé destiné à détecter une substance cible dans un flux gazeux comprenant les étapes ci-dessous consistant à :

> amener un flux gazeux comprenant la substance cible et de la vapeur d'eau à heurter un précurseur à électrolyte solide, de la forme d'un ligand organique et d'un ion métallique en mesure de former ensemble un complexe organométallique ;
> permettre au précurseur à électrolyte solide d'être hydraté, au moins partiellement, de manière à former un pont d'électrolyte entre une électrode active et une électrode auxiliaire ;
> appliquer un potentiel électrique à travers l'électrode active et l'électrode auxiliaire ;
> mesurer le courant circulant entre l'électrode active et l'électrode auxiliaire sous l'effet du potentiel appliqué ; et
> déterminer, à partir du flux de courant mesuré, une indication de la concentration de la substance cible dans le flux gazeux.

**18.** Procédé selon la revendication 17, dans lequel la substance cible est du dioxyde de carbone.

**19.** Procédé selon la revendication 17 ou 18, dans lequel une tension constante est appliquée à travers l'électrode active et l'électrode auxiliaire.

**20.** Procédé selon la revendication 17 ou 18, dans lequel une tension variable est appliquée à travers l'électrode active et l'électrode auxiliaire.

**21.** Procédé selon la revendication 20, dans lequel la tension variable alterne entre un potentiel de repos et un potentiel supérieur au potentiel seuil de réaction.

**22.** Procédé selon la revendication 21, dans lequel la tension est pulsée à une fréquence de l'ordre de 0,1 Hz à 20 kHz.

**23.** Procédé destiné à mesurer la concentration d'une substance cible dans l'air expiré d'un patient, le procédé comprenant les étapes ci-dessous consistant à :

> amener l'air expiré à heurter un précurseur à électrolyte solide de la forme d'un ligand organique et d'un ion métallique en mesure de former ensemble un complexe organométallique ;
> permettre au précurseur à électrolyte solide d'être hydraté, au moins partiellement, de manière à former un pont d'électrolyte entre une électrode active et une électrode auxiliaire ;
> appliquer un potentiel électrique à travers l'électrode active et l'électrode auxiliaire ;
> mesurer le courant circulant entre l'électrode active et l'électrode auxiliaire sous l'effet du potentiel appliqué ; et
> déterminer, à partir du flux de courant mesuré, une indication de la concentration de la substance cible dans le flux d'air expiré.

**24.** Procédé selon la revendication 23, dans lequel la substance cible est du dioxyde de carbone.

**25.** Procédé selon la revendication 23 ou 24, dans lequel le procédé est appliqué à un patient asthmatique.

**26.** Procédé selon l'une quelconque des revendications 23 à 25, dans lequel le mouvement respiratoire d'un patient est surveillé.

**27.** Système destiné à surveiller la composition d'un flux gazeux, comprenant :

un capteur selon l'une quelconque des revendications 1 à 16 ;
un microcontrôleur destiné à recevoir une sortie en provenance du capteur ; et
un écran d' affichage ;
dans lequel :

le microcontrôleur est programmé de manière à générer une image continue de la concentration d'une substance cible dans un flux gazeux en cours d'analyse sur l'écran d'affichage.

**28.** Système selon la revendication 27, dans lequel le capteur est apte à être exposé à la respiration d'un patient.

**29.** Système selon la revendication 27 ou 28, dans lequel la substance cible est du dioxyde de carbone.

Figure 1

1a

1b

1c

**Figure 2**

Figure 3

# Figure 4

# Figure 5

EP 1 782 052 B1

Vin

R2

**104**  **108**

R3

**100**

R1

OpAmp1

CE  WE

OpAmp2

Vout

**102**

Gnd

**106**

Gnd

## Figure 6

Figure 7

Figure 8

Figure 9

# Figure 10

EP 1 782 052 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 04001407 A
- US 4772863 A
- GB 2287543 A
- GB 2316178 A
- GB 2184549 A
- EP 0293230 A
- US 6454923 B

**Non-patent literature cited in the description**

- Handbook of Chemistry and Physics. Chemical Rubber Company, 1981